# EUROPEAN PATENT SPECIFICATION

(11) **EP 0 408 147 B1**
(45) Date of publication and mention of the grant of the patent: **14.05.1997**
(21) Application number: 90201877.9
(22) Date of filing: 10.07.1990
(51) Int. Cl.: G01N 35/00

(54) **Evaporation covers**
Verdampfungsabdeckungen
Couvercle contre l'évaporation

(30) Priority: 14.07.1989 US 380839
(43) Date of publication of application: 16.01.1991
(73) Proprietor: Johnson & Johnson Clinical Diagnostics, Inc., Rochester New York 14650 (US)
(72) Inventor: Przybylowicz, Catherine Salzman, Rochester, New York 14650-2201 (US); Jacobs, Merrit Nyles, Rochester, New York 14650-2201 (US); Douglas, Joseph Scott, Rochester, New York 14650-2201 (US)
(74) Representative: Mercer, Christopher Paul

(56) References cited:
- EP-A- 0 278 144
- US-A- 4 298 571
- US-A- 4 568 519
- US-A- 4 584 275
- US-A- 4 814 279

## Description

The invention concerns evaporation covers which are used in analyzers, especially such covers for test elements which have sample drops protruding therefrom.

Clinical analyzers have for years provided rapid and accurate tests using slide-like test elements, as described, for example, in US Patent Specification US-A-4298571. Incubators have been used in such analyzers, along with preheat stations, to control the temperature of the test element while a patient sample drop spotted thereon undergoes a chemical reaction to produce a detectable change. In such incubators, a test element is transferred from a spotting station to a preheat station, to an incubator, and then to a change-detecting station.

In most of the stations following the spotting station, a cover is used to contact the spotted test element to prevent significant evaporation of the sample. Such a cover is disclosed in US-A-4298571. Because some of the test elements are potentiometric types which have sample and/or reference drops protruding from the test element for a long time, the cover is grooved from side to side to allow clearance of the sample or drop. There is no need and no provision, however, in such covers, for the grooves to run from the exterior edge at which the test element enters, lengthwise along the direction of movement of the test element, since a piston is used to raise and lower the cover. Instead, the groove runs perpendicular to the direction of slide movement. Furthermore, the groove does not extend to any edge surface of the cover.

Covers with undercut grooves have been used for potentiometric test elements because the drops on such elements have protruded above the rest of the test element. Such protrusion after spotting has not been a significant problem with most colorimetric elements, at least, not the type also useful with the analyzer disclosed in US-A-4298571, since sample absorption is much more rapid in such colorimetric elements.

A problem has arisen, however, in the design of new analyzers which are to operate at higher throughput speeds to provide increased efficiency. Such new analyzers utilize a preheat station which receives colorimetric or rate test elements soon after spotting, e.g. 500ms afterwards, that the drop still protrudes above the test element. Conventional covers in stations following the spotting station, e.g. the preheat station, have been unsatisfactory since they are designed to fit with an undersurface which is in flush contact with the test element. Such flush fit means the drop is wiped onto the undersurface as the test element is advanced into the particular station. This in turn produces unacceptable contamination of the cover and loss of sample volume.

One attempt at solving this problem was to provide a longitudinal groove extending the full length of the cover. However, this was a failure due to the rapid evaporation which such a groove allowed to the drop.

It is therefore an object of the present invention to provide a solution which meets three competing goals, namely:
1) to provide a cover undersurface constructed in such a way that it does not wipe the drop of a colorimetric element,
2) to keep such a drop from experiencing significant evaporation, and
3) to minimize gaseous carryover.

In accordance with one aspect of the present invention, there is provided a clinical analyzer for use with slide-like test elements as defined in the claims, having a top surface and a drop-receiving surface below the top surface, the analyzer comprising a spotting station for placing a drop of patient sample onto a test element, and a plurality of further stations following the spotting station, at least one of the further stations including a cover having a front edge and a contact surface which is in contact with the top surface of each test element after it has been spotted, each contact surface having a groove therein adjacent to an exterior location, and transfer means for transferring each test element from the exterior location past the front edge of the cover to an interior location under the cover in a predetermined direction into each station, each test element being transferred whilst in contact with the cover;
characterized in that the groove,
a) has an axis of orientation which is parallel to said predetermined transferring direction,
b) has a clearance extending from said front edge and shape adequate to accommodate without contact any sample drop of a predetermined volume protruding above the top surface of the test element,
c) is closed at an end opposite to said front edge, and
d) has a width which is less than the width of a covered test element.

It is an advantageous feature of the invention that a cover is provided for already-spotted colorimetric or rate test elements which avoids contacting drops of sample not yet absorbed, without significant evaporation occurring.

Another advantageous feature is that such a cover can be constructed of a variety of materials and still prevent undesired carry-over of gaseous byproducts to subsequent test elements.

The present invention will now be described by way of example only with reference to the accompanying drawings in which:-
Figure 1 is a perspective view of the undersurface of a cover of the prior art;
Figure 2 is a fragmentary schematic view of an analyzer constructed in accordance with the present invention;
Figure 3 is a fragmentary sectioned view taken generally along line III-III of Figure 2; and
Figure 4 is a bottom plan view of the cover shown in Figure 3; and
Figure 5 is a cross section through line V of Figure 3.

The invention is particularly described regarding its use in a preheat station of an analyzer which is positioned between a spotting station and an incubator, the analyzer being used to process colorimetric-type test elements. In addition, it is useful with other types of test elements and in other stations which follow the spotting station, including the incubator, provided that the residence time of the test element in such other stations, and the flow rate of air in the vicinity of the cover, are such as to restrict significant evaporation. As used herein, evaporation is "significant" if the loss of fluid exceeds 0.5µl.

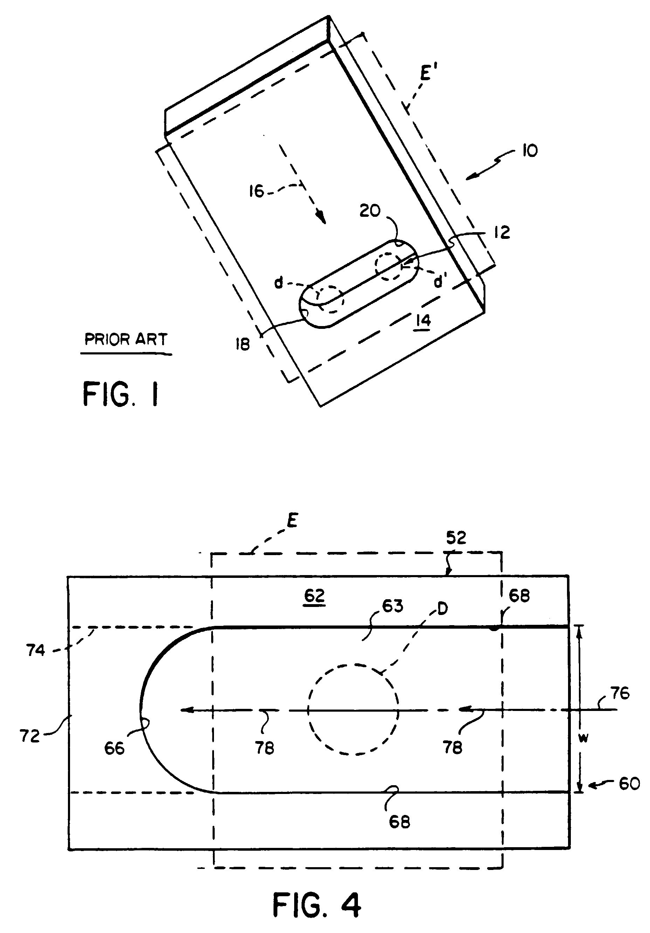

A cover constructed in accordance with US-A-4298571 is shown in Figure 1. The cover 10 has a groove 12 in undersurface 14 which extends transversely to the direction of movement of a test element under the cover 10, as shown by arrow 16. Groove 12 does not extend the full width, but is closed at both ends 18, 20. The depth of the groove 12 is such as to accommodate two drops (without touching). The drops are shown in phantom as d and d' on a potentiometric test element E', which is also in phantom.

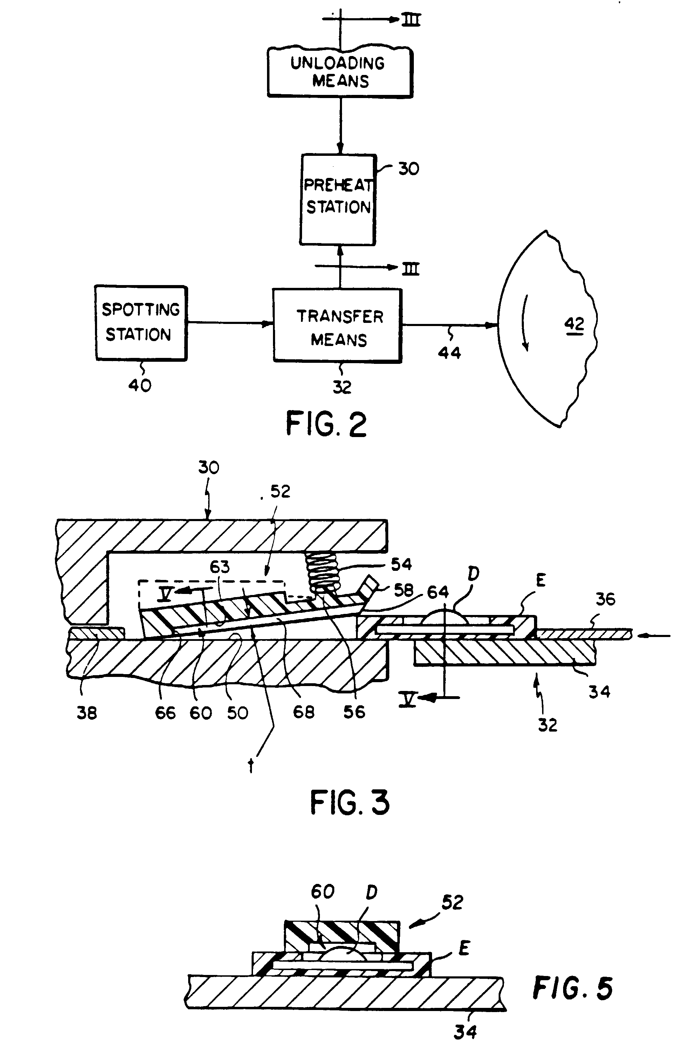

According to the present invention, an improved cover is preferably used in a preheat station 30 of an analyzer as shown in Figure 2. The preheat station 30 receives test elements from transfer means 32, which in turn receives each test element from a spotting station 40 and sends preheated elements on to an incubator 42, which can be any conventional incubator such as the rotating type illustrated. Transfer means 32, shown in Figure 3, preferably comprises a support platform 34 and a pusher blade 36 which pushes a spotted test element E into station 30 (also shown in phantom in Figure 4). A conventional pusher blade 38 can be used to return preheated elements to transfer means 32, where another mechanism (not shown) is effective to forward a test element to the incubator 42 as shown by arrow 44 in Figure 2. Alternatively, a more complicated finger arrangement (not shown) can be used to replace blades 36 and 38, as shown in Figure 3, so that the fingers both feed elements into preheat station 30, and return elements from that preheat station.

Preheat station 30 preferably comprises an element support surface 50, a cover 52, and biasing means 54 for biasing the cover down onto a test element E. Any biasing means will suffice, such as conventional compression springs. Cover 52 includes a boss 56 which cooperates with the biasing means 54, and a camming lip 58 to encourage a test element to be inserted between the cover 52 and support surface 50.

In accordance with one aspect of the invention, as shown in Figures 3 and 4, cover 52 comprises a groove 60 in undersurface 62 extending lengthwise from lower edge 64 of lip 58. In particular, edge 64 is the leading edge of undersurface 62, as shown in Figure 3, which is adjacent the exterior of the station 30 from whence comes a test element for insertion. However, groove 60 having bottom surface 63 is closed at end wall 66 which is interior of the station 30, leaving sidewalls 68 extending from end wall 66 to edge 64. The purpose of closed end 66 is to prevent circulating air from readily reaching a drop surrounded by sidewalls 18 and end wall 66, as shown in Figure 4, the position of a drop D being shown in phantom. The width w of the groove (Figure 4) as well as its depth t (Figure 3) are selected to keep the drop from contacting any part of the cover 52 for drop volumes no greater than 20µl, and preferably volumes of 10µl. Recognizing that some static electricity can attract the drop, t is most preferably at least about 0.254mm. The reason is that the clearance between bottom surface 63 of the groove and the top of a protruding drop is preferably at least 0.18mm, the drops having a volume no greater than about 12µl and thus a protrusion above the plane of the test element's exterior surface which is preferably about 0.17mm.

End wall 66 can be made quite thin, that is, can closely approach opposite edge 72 of cover 52. However, if groove 60 were to extend the full length as suggested by phantom lines 74 in Figure 4, it would provide too much evaporation of drop D, and therefore produce undesirable results.

Groove 60 has its long axis 76 oriented so as to be parallel to the direction of movement of the test element, as shown by arrows 78 in Figure 4.

Any material is useful for surfaces 62 and 63. Preferably, however, it is Teflon (Registered Trade Mark) or polyethylene. Surprisingly, Teflon is equally as good in this configuration in preventing carryover of SO₂ gas generated by phosphorus-testing test elements, compared to polyethylene. This result was found not to exist heretofore.

## Claims

1. A clinical analyzer for use with slide-like test elements (E) having a top surface and a drop-receiving surface below the top surface, the analyzer comprising a spotting station (40) for placing a drop of patient sample onto a test element (E), and a plurality of further stations (30) following the spotting station (40), at least one of the further stations (30) including a cover (52) having a front edge (64) and a contact surface (62) which is in contact with the top surface of each test element (E) after it has been spotted, each contact surface (62) having a groove (60) therein adjacent to an exterior location, and transfer means (32, 34, 36, 38) for transferring each test element (E) from the exterior location past the front edge (64) of the cover (52) to an interior location under the cover (52) in a predetermined direction into each station (30), each test element (E) being transferred whilst in contact with the cover (52);
characterized in that the groove (60)
a) has an axis (76) of orientation which is parallel to said predetermined transferring direction,
b) has a clearance extending from said front edge (64) and shape adequate to accommodate without contact any sample drop (D) of a predetermined volume protruding above the top surface of the test element (E),
c) is closed at an end (66) opposite to said front edge (64), and
d) has a width (w) which is less than the width of a covered test element (E).

2. An analyzer according to claim 1, wherein the station (30) having the cover (52) is a preheat station prior to an incubator.

3. An analyzer according to claim 1 or 2, wherein the clearance is at least about 0.18mm above the height a 10µl drop of liquid can occupy on a test element (E) before it is absorbed therein.

4. An analyzer according to claim 1 or 2, wherein the contact surface (62) comprises a material selected from TEFLON or polyethylene.

## Patentansprüche

1. Klinisches Analysegerät zur Verwendung mit objektträgerähnlichen Testelementen (A), die eine Oberseite und eine tropfenaufnehmende Fläche unterhalb der Oberseite aufweisen, wobei das Analysegerät eine Tropfenaufbringstation (40) zum Aufbringen eines Tropfens einer Patientenprobe auf ein Testelement (E), sowie eine Mehrzahl weiterer Stationen (30) hinter der Tropfenaufbringstation (40) umfaßt, mindestens eine der weiteren Stationen (30) eine Abdeckung (52) aufweist, die eine Vorderkante (64) und eine Kontaktfläche (62) hat, die, nachdem sie mit einem Tropfen versehen worden ist, mit der Oberseite jedes Testelementes (E) in Berührung steht, in jeder Kontaktfläche (62) eine Nut (60) in der Nähe einer äußeren Stelle vorhanden ist, und eine Überführungsvorrichtung (32, 34, 36, 38) zum Überführen jedes Testelementes (E) von einer äußeren Stelle an der Vorderkante (64) der Abdeckung (52) vorbei zu einer inneren Stelle unter der Abdeckung (52) in einer vorbestimmten Richtung in jede Station (30) vorgesehen ist, und jedes Testelement (E) in Kontakt mit der Abdeckung (52) überführt wird;
dadurch gekennzeichnet, daß die Nut (60)
a) eine Richtungsachse (76) hat, die parallel zu der vorbestimmten Überführungsrichtung gerichtet ist,
b) einen sich von der Vorderkante (64) erstreckenden Freiraum und eine Form hat, die für eine berührungslose Unterbringung irgendeines Probentropfens (D) vorbestimmten Volumens hinreichend ist, der über die Oberseite des Testelementes (E) vorragt,
c) an einem der Vorderkante (64) gegenüberliegenden Ende geschlossen ist, und
d) eine Breite (w) hat, die geringer als die Breite eines abgedeckten Testelementes (E) ist.

2. Analysegerät nach Anspruch 1, wobei die Station (30) mit dem Deckel (52) eine Vorwärmstation vor einem Inkubator ist.

3. Analysegerät nach Anspruch 1 oder 2, wobei der Freiraum mindestens etwa 0,18 mm höher als die Höhe ist, die ein 10 µl Flüssigkeitstropfen auf einem Testelement (E) einnehmen kann, bevor er darin absorbiert wird.

4. Analysegerät nach Anspruch 1 oder 2, wobei die Kontaktfläche (62) ein Material umfaßt, das aus Teflon oder Polyethylen ausgewählt ist.

## Revendications

1. appareil d'analyse clinique, pour utilisation avec des éléments d'essai sous forme de plaque coulissante (E) ayant une surface supérieure et une surface de réception de gouttes en dessous de la surface supérieure, l'appareil d'analyse comprenant un poste de déposition (40) pour placer une goutte de prélèvement d'un patient sur un élément d'essai (E), et plusieurs postes supplémentaires (30) à la suite du poste de déposition (40), au moins un des postes supplémentaires (30) comprenant un couvercle (52) ayant un bord avant (64) et une surface de contact (62) qui est en contact avec la surface supérieure de chaque élément d'essai (E) après qu'on y a déposé un prélèvement, chaque surface du contact (62) ayant à l'intérieur une rainure (60) adjacente à un emplacement extérieur, et des moyens de transfert (32, 34, 36, 38) pour transférer chaque élément d'essai (E) de l'emplacement extérieur, au-delà du bord avant (64) du couvercle (52) vers un emplacement intérieur, sous le couvercle (52) dans une direction prédéterminée dans chaque poste (30), chaque élément d'essai (E) étant transféré tout en étant en contact avec le couvercle (52) ;
caractérisé en ce que la rainure (60)
a) a un axe (76) d'orientation qui est parallèle à ladite direction de transfert prédéterminée,
b) comporte un espacement s'étendant depuis ledit bord avant (64) et une forme adéquate pour loger sans contact n'importe quelle goutte de prélèvement (D) d'un volume prédéterminé formant saillie au-dessus de la surface supérieure de l'élément d'essai (E),
c) est fermée à une extrémité (66) opposée audit bord avant (64), et
d) a une largeur (w) qui est inférieure la largeur d'un élément d'essai recouvert (E).

2. Appareil d'analyse selon la revendication 1, dans lequel le poste (30) ayant le couvercle (52) est un poste de préchauffage avant un incubateur.

3. Appareil d'analyse selon les revendications 1 ou 2, dans lequel l'espacement est d'au moins environ 0,18 mm au-dessus de la hauteur qu'une goutte de liquide de 10 l peut occuper sur un élément d'essai (E) avant qu'elle soit absorbée dedans.

4. Appareil d'analyse selon les revendications 1 ou 2, dans lequel le surface du contact (62) est constituée d'une matière choisie à partir de TEFLON ou de polyéthylène.
